# EUROPEAN PATENT SPECIFICATION

(11) **EP 2 007 678 B1**
(45) Date of publication and mention of the grant of the patent: **08.05.2013**
(21) Application number: 07712508.6
(22) Date of filing: 12.03.2007
(51) Int. Cl.: C01B 33/18, C01B 37/00, C01B 39/08, C07D 301/12, B01J 29/04

(54) **PROCESS FOR THE PREPARATION OF A SILICON-TITANIUM MIXED OXIDE POWDER CONTAINING DISPERSION**
PROZESS ZUR HERSTELLUNG EINER SILICIUM-TITAN-MISCHOXID-PULVER ENTHALTENDEN DISPERSION
PROCÉDÉ DE PRÉPARATION D'UNE DISPERSION CONTENANT UNE POUDRE D'OXYDE MIXTE DE SILICIUM-TITANE

(30) Priority: 15.04.2006 DE 102006017701
(43) Date of publication of application: 31.12.2008
(60) Divisional application: 13155806.6
(73) Proprietor: Evonik Degussa GmbH, 45128 Essen (DE)
(72) Inventor: SCHUMACHER, Kai, 65719 Hofheim (DE); MÖRTERS, Martin, 79618 Rheinfelden (DE); MANGOLD, Helmut, 63517 Rodenbach (DE); HASENZAHL, Steffen, 63454 Hanau (DE)
(86) International application number: PCT/EP2007/052284
(87) International publication number: WO 2007/118739

(56) References cited:
- EP-A1- 0 814 058
- US-A1- 2003 129 153

## Description

The invention relates to a dispersion comprising the pyrogenic silicon-titanium mixed oxide powder.

The use of silicon-titanium mixed oxide powders for the preparation of titanium-containing zeolites is known from EP-A-814058. Titanium-containing zeolites are efficient catalysts for the oxidation of olefins using hydrogen peroxide. They are obtained by hydrothermal synthesis starting from silicon-titanium mixed oxide powders in the presence of a template. In EP-A-814058, it is disclosed that pyrogenic silicon-titanium mixed oxides having a silicon dioxide content of 75 to 99.9% by weight and a titanium dioxide content of 0.1 to 25% by weight can be employed for this. A composition which contains from 90 to 99.5% by weight of silicon dioxide and 0.5 to 5% by weight of titanium dioxide is particularly advantageous. As templates, amines, ammonium compounds or alkali/alkaline earth metal hydroxides can be employed.

A disadvantage of the process disclosed in EP-A-814058 is the long reaction time which is necessary for the reaction of the silicon-titanium mixed oxide in the presence of the template. Furthermore, not all titanium-containing zeolites obtained according to EP-A-814058 show adequate catalytic activity.

It is the object of the invention to provide an alternative process for the preparation of a dispersion comprising a silicon-titanium mixed oxide.

The invention relates to a process for the preparation of a the dispersion comprising a pyrogenic silicon-titanium mixed oxide powder, having
- a the BET surface area is 200 to 400 m²/g,
- a the silicon dioxide content is 97.0 ± 1.5% by weight,
- a the titanium dioxide content is 3.5 ± 1.0% by weight and
- wherein the sum of silicon dioxide content and titanium dioxide content is greater than 99.7% by weight, the percentages by weight relating to the total amount of the powder,
   comprising the steps:
- water, which, if the silicon-titanium mixed oxide powder introduced later leads to a pH of the aqueous phase of <2 or >4, is adjusted by addition of acids or bases to pH of 2 to 4, is recycled from a receiver by means of a rotor/stator machine, and
- an amount of silicon-titanium mixed oxide powder is introduced continuously or batchwise by means of a filling device and with the rotor/stator machine running into the shear zone between the slots of the rotor teeth and of the stator slots such that a pre-dispersion having a solids content of 20 to 40% by weight results, and
- after all the silicon-titanium mixed oxide powder has been added, the filling device is closed and the predispersion is sheared further such that the shear rate lies in the range between 10 000 and 40 000 s-1, and
- if appropriate water and a basic, quaternary ammonium compound are subsequently added with retention of the dispersion conditions.

Pyrogenic is to be understood as meaning metal mixed oxide particles obtained by flame oxidation and/or flame hydrolysis. In this process, oxidizable and/or hydrolysable starting substances are as a rule oxidized or hydrolysed in a hydrogen-oxygen flame. The metal mixed oxide particles according to the invention are as far as possible pore-free and have free hydroxyl groups on the surface. They are present in the form of aggregated primary particles.

It has been shown that a high BET surface area markedly reduces the period of time for the preparation of a titanium-containing zeolite from the silicon-titanium mixed oxide powder.

A silicon-titanium mixed oxide powder having a BET surface area of 250 to 350 m²/g is preferred and particularly preferably one of 300 ± 30 m²/g.

Furthermore, a silicon-titanium mixed oxide powder having a silicon dioxide content of 97.0 ± 1.0% by weight and a titanium dioxide content of 3.5 ± 0.75% by weight is preferred where the sum of silicon dioxide content and titanium dioxide content is greater than 99.9% by weight. A silicon-titanium mixed oxide powder having a silicon dioxide content of 97.0 ± 0.5% by weight and a titanium dioxide content of 3.5 ± 0.5% by weight is particularly preferred where the sum of silicon dioxide content and titanium dioxide content is greater than 99.9% by weight.

The sum of silicon dioxide content and titanium dioxide content in the powder is greater than 99.7% by weight and preferably greater than 99.9% by weight. The content of the metals Al, Ca, Co, Fe, K, Na, Ni and Zn is preferably less than 50 ppm each and particularly preferably less than 25 ppm each. The content of chloride is preferably less than 700 ppm. It has proved advantageous for the preparation of titanium-containing zeolites if the contents of these metals and chloride do not exceed these values. These impurities can originate from the required substances and/or can be caused due to the process.

The silicon-titanium mixed oxide powder can be prepared by a process in which
- 97.0 ± 1.5 parts by weight calculated as SiO₂ of a silicon chloride and 3.5 ± 1.0 parts by weight calculated as TiO₂ of a titanium chloride are evaporated, the vapours are taken to a mixing chamber, hydrogen and primary air are taken to the mixing chamber separately therefrom,
- the mixture of the vapours of silicon chloride and titanium chloride, hydrogen-containing combustible gas and primary air is subsequently ignited in a burner and the flame is burned into a reaction chamber,
- secondary air is additionally introduced into the reaction chamber, the solid is subsequently separated from gaseous substances, and
- the solid is subsequently freed as far as possible from halide-containing substances by treatment with steam at temperatures of 250 to 700°C
- the amount of the required substances consisting of silicon chloride, titanium chloride, combustible gas, primary air and secondary air being chosen such that an adiabatic flame temperature T_{ad} results, for which the following is true:
   900°C < T_{ad} < 1200°C,
with
T_{ad} = temperature of required substances + sum of the reaction enthalpies of the partial reactions/heat capacity of the substances which leave the reaction chamber, comprising silicon-titanium mixed oxide, water, hydrogen chloride, if appropriate carbon dioxide, oxygen, nitrogen, and if appropriate of the carrier gas if this is not air or nitrogen, the specific heat capacity of these substances at 1000°C being used as a basis.

The specific heat capacities can be determined, for example, with the aid of the VDI Wärmeatlas [VDI heat atlas] (Chapter 7.1 to 7.3 and 3.7, 8th Edition).

The reaction of the silicon chlorides and titanium chlorides in the presence of oxygen and of a combustible gas yields silicon-titanium mixed oxide, water, hydrochloric acid and, in the case of carbon-containing silicon and/or titanium compounds and/or carbon-containing combustible gases, carbon dioxide. The reaction enthalpies of these reactions can be calculated by means of standard works known to the person skilled in the art.

In Table 1, some selected values of reaction enthalpies of the reaction of silicon halides and titanium tetrachloride in the presence of hydrogen and oxygen are given.

Methyltrichlorosilane (MTCS, CH₃SiCl₃), trichlorosilane (TCS, SiHCl₃) and/or dichlorosilane (DCS, SiH₂Cl₂) and titanium tetrachloride can particularly preferably be employed.

**Table 1: Reaction enthalpies**

| | KJ/mol |
|---|---|
| H₂ | -241.8 |
| SiCl₄ | -620.1 |
| SiHCl₃ | -659.4 |
| SiH₂Cl₂ | -712.3 |
| C₃H₇SiCl₃ | -2700.2 |
| CH₃SiCl₃ | -928.3 |
| (CH₃)₃SiCl | -2733.8 |
| TiCl₄ | -553.4 |

Suitable combustible gases are hydrogen, methane, ethane, propane and/or natural gas, hydrogen being preferred.

It can further be advantageous if the exit velocity of the reaction mixture from the mixing chamber to the reaction space is 10 to 80 m/s.

The vapours of the silicon chloride and of the titanium chloride can also be taken to the mixing chamber, in mixed or separate form, by means of a carrier gas.

The required substances combustible gas, primary air and/or secondary air can be introduced in preheated form. A suitable temperature range is 50 to 400°C.

Furthermore, primary and/or secondary air can be enriched with oxygen.

Preferably, the process according to the invention can be carried out such that SiCl₄ is employed as silicon halide, TiCl₄ is employed as titanium halide and the adiabatic flame temperature T_{ad} = 1050 ± 50°C.

The product of the process according to the invention is a dispersion which comprises the silicon-titanium mixed oxide powder according to the invention and water.

The average aggregate diameter of the silicon-titanium mixed oxide particles in the dispersion is preferably less than 200 nm and particularly preferably less than 100 nm.

Preferably, the following is true for the dispersion according to the invention: 10 ≤ mol of water/mol of silicon-titanium mixed oxide ≤ 20. Particularly preferably, the range is 12 ≤ mol of water/mol of silicon-titanium mixed oxide ≤ 17.

Furthermore, a dispersion can be preferred which additionally contains a basic, quaternary ammonium compound. Dispersions are particularly preferred which contain tetraalkylammonium hydroxides such as, for example, tetraethylammonium hydroxide, tetra-n-propylammonium hydroxide and/or tetra-n-butylammonium hydroxide.

The content of quaternary, basic ammonium compound in the dispersion is not limited. If the dispersion is to be stored for a relatively long time, it can be advantageous to add to it only a part of the amount of the dispersion necessary for the preparation of a titanium-containing zeolite. Preferably, the quaternary, basic ammonium compound can be added in such an amount that a pH of 9 to 11, in particular 9.5 to 10.5, results. The dispersion shows good stability in this pH range.

If the dispersion is to be employed, for example, immediately after its preparation for the preparation of a titanium-containing zeolite, the dispersion can already also contain the total amount of quaternary, basic ammonium compound. Preferably, the following is then true: 0.12 ≤ mol of ammonium compound/mol of silicon-titanium mixed oxide < 0.20, 0.13 ≤ mol of ammonium compound/mol of silicon-titanium mixed oxide ≤ 0.17 being particularly preferred.

For the preparation of a titanium-containing zeolite, the silicon-titanium mixed oxide powder and a basic, quaternary ammonium compound can be treated in an aqueous medium at a temperature of 150 to 220°C for a period of less than 12 hours.

Preferably, the process is carried out such that the following is true: 10 ≤ mol of water/mol of silicon-titanium mixed oxide ≤ 20. Particularly preferably, the range is 12 ≤ mol of water/mol of silicon-titanium mixed oxide ≤ 17.

It is furthermore advantageous to carry out the process such that the following is true: 0.12 ≤ mol of ammonium compound/mol of silicon-titanium mixed oxide < 0.20. Particularly preferably, the range is 0.13 ≤ mol of ammonium compound/mol of silicon-titanium mixed oxide ≤ 0.16.

As basic, quaternary ammonium compounds, tetraalkylammonium hydroxides such as, for example, tetraethylammonium hydroxide, tetra-n-propylammonium hydroxide and/or tetra-n-butylammonium hydroxide are particularly preferred.

Basic, quaternary ammonium compounds are used as templates which determine the crystal structure by incorporation into the crystal lattice. Tetra-n-propylammonium hydroxide is preferably employed for the preparation of titanium silicalite-1 (MFI structure), tetra-n-butylammonium hydroxide for the preparation of titanium silicalite-2 (MEL structure) and tetraethylammonium hydroxide for the preparation of titanium β-zeolites (BEA crystal structure).

For the preparation of a titanium-containing zeolite, in which the silicon-titanium mixed oxide powder dispersion, if appropriate with further addition of a basic, quaternary ammonium compound, can be treated at a temperature of 150 to 220°C for a period of less than 12 hours.

Under the specified conditions of the process, the crystallization time is conventionally less than 12 hours. The crystals are separated by filtering, centrifuging or decanting and washed with a suitable washing liquid, preferably water. The crystals are then dried if needed and calcined at a temperature between 400°C and 1000°C, preferably between 500°C and 750°C in order to remove the template.

The particle fineness of less than 200 nm in the dispersion leads to rapid dissolution of the particles and formation of the titanium-containing zeolite.

The titanium-containing zeolite is obtainable by the process starting from silicon-titanium mixed oxide powder.

The titanium-containing zeolite is obtainable by the process starting from the dispersion comprising silicon-titanium mixed oxide powder.

Both titanium-containing zeolites are obtained in powder form. For their use as an oxidation catalyst, they are converted if needed to a form suitable for use, e.g. to micropellets, spheres, tablets, solid cylinders, hollow cylinders or honeycombs, using known methods for the creation of pulverulent catalysts, such as, for example, pelletization, spray drying, spray pelletization or extrusion.

The titanium-containing zeolites can be used as catalysts in oxidation reactions with hydrogen peroxide. In particular, they can be used as catalysts in the epoxidation of olefins with the aid of aqueous hydrogen peroxide in a water-miscible solvent.

### Examples:

Required materials: The required materials silicon tetrachloride and titanium tetrachloride of Examples 1 to 5 have contents of Na, K, Fe, Co, Ni, Al, Ca and Zn of < 50 ppm.

### Examples 1 to 5: Titanium-silicon mixed oxide powder

Example 1: 5.15 kg/h of silicon tetrachloride and 0.15 kg/h of titanium tetrachloride are evaporated. The vapours are taken to a mixing chamber by means of 15 Nm³/h of nitrogen as a carrier gas. Separately therefrom, 2 Nm³/h of hydrogen and 8 Nm³/h of primary air are introduced into the mixing chamber. The reaction mixture is fed to a burner and ignited in a central tube. The flame burns here in a water-cooled flame tube. 15 Nm³/h of secondary air are additionally introduced into the reaction space. The resulting powder is separated in a filter connected in series and subsequently treated with water vapour at 520°C in countercurrent.

Examples 2-5 are carried out analogously to Example 1 using the amounts listed in the table.

The substance parameters of the powders obtained are summarized in the table.

In all examples, the content of Na is <10 ppm, K <10 ppm, Fe ≤1 ppm, Co <1 ppm, Ni <1 ppm, Al <10 ppm, Ca <10 ppm, Zn <10 ppm.

**Table: Required substances and amounts, analytical values of the silicon-titanium mixed oxide powders**

| **Example** | | **1** | **2** | **3** | **4** | **5** |
|---|---|---|---|---|---|---|
| **SiCl₄** | kg/h | 5.15 | 8.0 | 8.0 | 5.15 | 5.15 |
| **TiCl₄** | kg/h | 0.15 | 0.21 | 0.21 | 0.15 | 0.15 |
| **H₂ core** | Nm³/h | 2.0 | 3.0 | 3.4 | 2.10 | 3.50 |
| **H₂ jacket** | Nm³/h | 1.0 | 0.5 | 0.5 | 1.0 | 1.0 |
| **Primary air** | Nm³/h | 8.0 | 10.7 | 10.0 | 12.5 | 10.0 |
| **Secondary air** | Nm³/h | 15.0 | 15.0 | 15.0 | 15.0 | 15.0 |
| **T_{ad}** | °C | 1026 | 1059 | 1160 | 930 | 1275 |
| **v_{Br}** | m/s | 32 | 30 | 21 | 33 | 31 |
| **BET** | m²/g | 312 | 315 | 203 | 375 | 80 |
| **SiO₂** | % by wt | 96.4 | 96.8 | 96.4 | 96.6 | 96.6 |
| **TiO₂** | % by wt | 3.4 | 3.0 | 3.5 | 3.3 | 3.4 |

### Example 6: Preparation of a dispersion (according to the invention)

32.5 kg of completely demineralized water are initially introduced into a 100 1 stainless steel make-up vessel. Subsequently, with the aid of the suction nozzle of the Ystral Conti-TDS 4 (stator slots: 6 mm ring and 1 mm ring, rotor/stator distance about 1 mm), 17.5 kg of the silicon-titanium mixed oxide powder from Example 1 are drawn in under shear conditions. After completion of the drawing-in, the suction nozzle is closed and the 35 per cent by weight pre-dispersion is subsequently additionally sheared at 3000 rpm for 10 min. Undesired warming of the dispersion due to the high energy input is countered by a heat exchanger and the temperature increase is restricted to a maximum of 40°C. Due to the acidic character of the pyrogenically prepared silicon-titanium mixed oxide powder, the pH of the dispersion is about 3.6.

Subsequently, 28.6 kg of completely demineralized water are added and a pH of 10.0 is rapidly adjusted with intensive shearing and thorough mixing using 1.0 kg of tetra-n-propylammonium hydroxide solution (40% by weight in water).

The dispersion has the following values:
water/silicon-titanium mixed oxide 11.7
average aggregate diameter 92 nm (determined with Horiba LA 910)

### Example 7: Preparation of a titanium-containing zeolite starting from silicon-titanium mixed oxide powder

137.0 g of a tetra-n-propylammonium hydroxide solution (40% by weight in water) and 434.2 g of deionized water are initially introduced into a polyethylene beaker and 111.1 g of the pyrogenic silicon-titanium mixed oxide powder from Example 1 are incorporated with intensive stirring. The resulting gel is initially aged for 2 hours at 80°C with intensive stirring and subsequently crystallized in an autoclave at 180°C for 10 hours. The solid obtained is separated from the mother liquor by centrifuging, washed three times with 250 ml each of deionized water, dried at 90°C and calcined in an air atmosphere for 4 hours at 550°C.
Water/silicon-titanium mixed oxide 13.1
Tetrapropylammonium hydroxide/silicon-titanium mixed oxide 0.15

Example 8 is carried out analogously to Example 7 but using the silicon-titanium mixed oxide powder from Example 5. The incorporation of the powder manifestly needs more time than in Example 7.

### Example 9: Preparation of a titanium-containing zeolite starting from a dispersion comprising silicon-titanium mixed oxide powder

505 g of the dispersion from Example 6, 46.7 g of deionized H₂O and 130.6 g of a tetra-n-propylammonium hydroxide solution (40% by weight in water) are initially introduced into a polyethylene beaker and initially aged for four hours at 80°C with stirring and subsequently crystallized in an autoclave at 180°C for 10 hours. The solid obtained is separated from the mother liquor by centrifuging, washed three times with 250 ml each of deionized water, dried at 90°C and calcined in an air atmosphere for four hours at 550°C.
Water/silicon-titanium mixed oxide 13.2
Tetrapropylammonium hydroxide/silicon-titanium mixed oxide 0.14

The X-ray diffractogram of the crystals obtained from Examples 7 to 9 shows the diffraction pattern typical for the MFI structure; the IR spectrum shows the characteristic band at 960 cm⁻¹. The UV-vis spectrum shows that the sample is free of titanium dioxide and titanates.

In the epoxidation of propylene using aqueous hydrogen peroxide solution, the following is true for the catalytic activity of the titanium silicalites obtained from Examples 7, 8 and 9: 9 > 7 >> 8.

## Claims

1. Process for the preparation of a dispersion comprising a pyrogenic silicon-titanium mixed oxide powder, having
- a the BET surface area is 200 to 400 m²/g,
- a the silicon dioxide content is 97.0 ± 1.5% by weight,
- a the titanium dioxide content is 3.5 ± 1.0% by weight and
- wherein the sum of silicon dioxide content and titanium dioxide content is greater than 99.7% by weight, the percentages by weight relating to the total amount of the powder,
comprising the steps:
- water, which, if the silicon-titanium mixed oxide powder introduced later leads to a pH of the aqueous phase of <2 or >4, is adjusted by addition of acids or bases to pH of 2 to 4, is recycled from a receiver by means of a rotor/stator machine, and
- an amount of silicon-titanium mixed oxide powder is introduced continuously or batchwise by means of a filling device and with the rotor/stator machine running into the shear zone between the slots of the rotor teeth and of the stator slots such that a pre-dispersion having a solids content of 20 to 40% by weight results, and
- after all the silicon-titanium mixed oxide powder has been added, the filling device is closed and the predispersion is sheared further such that the shear rate lies in the range between 10 000 and 40 000 s-1, and
- if appropriate water and a basic, quaternary ammonium compound are subsequently added with retention of the dispersion conditions.

## Patentansprüche

1. Verfahren zur Herstellung einer Dispersion umfassend ein pyrogenes Silicium-Titan-Mischoxidpulver, wobei
- die BET-Oberfläche 200 bis 400 m²/g beträgt,
- der Siliciumdioxid-Anteil 97,0 ± 1,5 Gew.-% beträgt,
- der Titandioxid-Anteil 3,5 ± 1,0 Gew.-% beträgt und
- die Summe aus Siliciumdioxid-Anteil und Titandioxid-Anteil größer als 99,7 Gew.-% ist,
wobei sich alle Gewichtsprozentangaben auf die Gesamtmenge des Pulvers beziehen, umfassend die Schritte:
- Wasser, welches, falls das später eingebrachte Silicium-Titan-Mischoxidpulver zu einem pH-Wert der wässerigen Phase von <2 oder >4 führt, durch Zugabe von Säuren oder Basen auf pH-Werte von 2 bis 4 eingestellt ist, wird aus einer Vorlage über eine Rotor-/Statormaschine im Kreis geführt, und
- über eine Einfüllvorrichtung wird kontinuierlich oder diskontinuierlich und bei laufender Rotor-/Statormaschine eine solche Menge Silicium-Titan-Mischoxidpulver in die Scherzone zwischen den Schlitzen der Rotorzähne und der Statorschlitze eingebracht, dass eine Vordispersion mit einem Feststoffgehalt von 20 bis 40 Gew.-% resultiert, und
- man nachdem alles Silicium-Titan-Mischoxidpulver zugegeben ist, die Einfüllvorrichtung schließt und so weiterschert, dass die Scherrate im Bereich zwischen 10,000 bis 40,000 s⁻¹ liegt, und
- gegebenenfalls anschließend unter Beibehalten der Dispergierbedingungen Wasser und eine basische, quaternäre Ammoniumverbindung hinzugibt.

## Revendications

1. Procédé de préparation d'une dispersion comprenant une poudre pyrogène d'oxyde mixte de silicium-titane ayant :
- une surface spécifique BET de 200 à 400 m²/g,
- une teneur en dioxyde de silicium de 97,0 ± 1,5 % en poids,
- une teneur en dioxyde de titane de 3,5 ± 1,0 % en poids,
- dans laquelle la somme de la teneur en dioxyde de silicium et de la teneur en dioxyde de titane est supérieure à 99,7 % en poids, les pourcentages en poids se rapportant à la quantité totale de la poudre, comprenant les étapes suivantes :
- de l'eau, qui si la poudre d'oxyde mixte de silicium-titane introduite ultérieurement conduit à un pH de la phase aqueuse < 2 ou > 4 est ajustée par addition d'acides ou de bases à un pH de 2 à 4, est recyclée depuis un réservoir au moyen d'une machine à rotor/stator, et
- une quantité de poudre d'oxyde mixte de silicium-titane est introduite de façon continue ou discontinue au moyen d'un dispositif de remplissage et avec la machine à rotor/stator en fonctionnement dans la zone de cisaillement entre les dents du rotor et les fentes du stator de telle sorte qu'une prédispersion ayant une teneur en matières solides de 20 à 40 % en poids en résulte, et
- après que toute la poudre d'oxyde mixte de silicium-titane a été ajoutée, le dispositif de remplissage est fermé et la prédispersion est encore cisaillée de telle sorte que la vitesse de cisaillement se situe dans la gamme entre 10 000 et 40 000 s-1, et
- lorsqu'il convient, de l'eau et un composé d'ammonium quaternaire basique sont ajoutés par la suite avec maintien des conditions de dispersion.
